# EUROPÄISCHE PATENTSCHRIFT

(11) **EP 2 371 418 B1**
(45) Veröffentlichungstag und Bekanntmachung des Hinweises auf die Patenterteilung: **03.11.2021**
(21) Anmeldenummer: 11156793.9
(22) Anmeldetag: 03.03.2011
(51) Int. Cl.: A61N 1/375

(54) **Elektrische Durchführung für elektromedizinische Implantate**
Electrical feedthrough for electromedical implants
Conduite électrique pour implants électromédicaux

(30) Priorität: 29.03.2010 US 318405 P
(43) Veröffentlichungstag der Anmeldung: 05.10.2011
(73) Patentinhaber: BIOTRONIK SE & Co. KG, 12359 Berlin (DE)
(72) Erfinder: Teske, Josef, 96103, Hallstadt (DE)
(74) Vertreter: Biotronik Corporate Services SE

(56) Entgegenhaltungen:
- WO-A1-03/073450
- US-A- 5 870 272
- US-A1- 2002 027 484
- US-A1- 2005 007 718
- US-A1- 2007 112 398
- US-B1- 6 414 835

## Beschreibung

Die Erfindung betrifft eine elektrische Durchführung für elektromedizinische Implantate wie implantierbare Herzschrittmacher, Defibrillatoren, Cardioverter, Nerven- und Himstimulatoren, Hörgeräte, implantierbare Medikamentenpumpen oder sonstige elektrische aktive Implantate, welche ein hermetisch dichtes Gehäuse umfassen, sowie Batterien mit hermetisch dichtem Gehäuse für diese elektronischen Implantate.

Üblicherweise weisen solche Durchführungen einen Flansch auf, mit dem sie in einer Gehäusewand des elektromedizinischen Implantats eingesetzt sind, vorzugsweise durch ein thermisches Fügeverfahren wie Schweißen oder Löten. Im Gehäuse befindet sich eine Einrichtung unter anderem mit einer Platine, welche in der Lage ist, elektrische Signale zu verarbeiten oder zu senden. Die Durchführung weist mindestens eine Durchführungshülse auf, einem die mindestens eine Durchführungshülse umgebenden Flansch, in der mindestens ein Anschlussstift sitzt, welcher von der mindestens einen Durchführungshülse umgeben ist. Der Anschlussstift erstreckt sich durch den Flansch und die Durchführungshülse hindurch von einem inneren Ende im Inneren des Gehäuses zu einem äußeren Ende, welches außerhalb des hermetisch dichten Gehäuses liegt. In der Regel sind der Anschlussstift mit der mindestens einen Durchführungshülse und/oder die mindestens eine Durchführungshülse mit dem Flansch mittels einer Lotverbindung, vorzugsweise bei Verwendung von metallisch beschichtete Durchführungshülsen (beispielsweise eine Niob-Beschichtung) mittels eines Goldlotes oder bei Verwendung unbeschichteter Durchführungshülsen mittels eines biokompatiblen Glaslotes ((Typ 8625 der Firma Schott), verbunden. Im Hinblick darauf, dass das äußere Ende der Anschlusselektrode bei einem medizinischen Implantat mit dem das Implantat umgebenden Körpergewebe in Kontakt kommen kann, sind die Anschlussstifte in der Regel aus einem biokompatiblen Material, wie beispielsweise Niob (Nb), Platin (Pt), Iridium (Ir), Platin/Iridium-Legierungen (Pt/Ir), Tantal (Ta), Titan (Ti), Zirkonium (Zr), Hafnium (Hf), medizinischer Edelstahl (z.B. 316L) oder Legierungen aus diesen Materialien gefertigt. Möglich sind als Materialien für den Anschlussstift auch Fe-Ni, FeNiCo, FeCr, Molybdän (Mo), Wolfram (W), Chrom (Cr), FeCr, Vanadium (V), Aluminium (Al) oder anderen Legierungen aus diesen Materialien.

Die Durchführungshülse ist üblicherweise aus einem Keramikmaterial wie beispielsweise Aluminiumoxid (Al₂O₃) hergestellt. Vor Allem bei Anschlussstiften aus Nb, Ta oder Ti besteht das Problem, dass zur Herstellung sicherer, niederohmiger, mechanisch stabiler und langlebiger elektrischer Kontakte mit den genannten biokompatiblen Anschlussstiften nur Schweißverfahren in Frage kommen, um eine Verbindung mit anderen Leitern herzustellen, wie beispielsweise mit den Anschlussleitungen oder mit der auf der Schaltung im Inneren des Implantates angelegten Geräteelektronik. Die erforderlichen hohen Temperaturen des Schweißvorganges können jedoch beispielsweise Metalldämpfe und Schweißspritzer erzeugen, die die elektrische Isolationsfähigkeit der Keramiken beeinträchtigen bzw. die Platine schädigen und bedürfen deshalb häufig zusätzlicher, Schutzmaßnahmen. Auch ist durch diese Eigenschaft das im Elektroniksektor bekannte und produktionstechnisch einfache und rationelle Reflow-Löten bei einem solchen Anschlussstift also nicht möglich oder nicht ohne weiteres einsetzbar.

Bei genannten Keramikdurchführungen mit Platin/Iridium-Anschlussstiften ist bekannt, dass sie ohne spezielle Schutzvorkehrungen Probleme mit der Auflösung der metallischen Beschichtung der Durchführungshülsen bei der Verlötung mit Goldlot zeigen sowie eine schlechte Benetzbarkeit der Pt/Ir-Oberflächen mit Weichlot aufweisen. Im Ergebnis ist das erwähnte Reflow-Löten unsicher.

Grundsätzlich ist die Beschichtung der Stiftoberflächen bei Nb-, Ta- oder Ti-Anschlussstiften auf der Innenseite zur leichten Benetzbarkeit mit Weichlot für die Anbindung an die interne Elektronik entweder gar nicht möglich oder nur unter erhöhtem Aufwand, indem beispielsweise auf schweißtechnischem oder plasmaphysikalischem Wege metallische, weichlötbare Beschichtungen aufgebracht werden. Weichlötbare Oberflächen oder Beschichtungen auf Ni, Ta oder Ti, die mit Hilfe von Flussmitteln oder auf galvanischem Wege aufgebracht werden, sind bisher unbekannt.

Aus der US 5,870,272 A ist eine Durchführung für implantierbare medizinische Geräte mit einem integrierten kapazitiven Filter bekannt, bei der die elektrische Kontaktierung und mechanische Verbindung zwischen einem beispielsweise aus Nb bestehenden Pin zu einem inneren Kontaktpad unter Zwischenschaltung des kapazitiven Filters über eine aufwändige, mehrstufige Lötanordnung mit Hart- und Weichlot hergestellt wird. Diese Konstruktionsweise ist für Durchführungen mit einfachen Anschlusselektroden zu komplex, bei einer entsprechenden Auslegung der Durchführung wäre eine rationelle Fertigung nicht möglich.

Weiterhin ist aus der US2005/0007718 A1 eine Durchführung für eine aktive implantierbare medizinische Vorrichtung bekannt, bei der eine Drahtbondkappe auf einen Anschlussdraht der Durchführung aufgesetzt ist.

Der Erfindung liegt die Aufgabe zugrunde, eine hermetisch dichte Durchführung für Anschlusselektroden aus einem körperverträglichen Material zur Implantat-Außenseite hin anzugeben, die auf herstellungstechnisch einfache Weise an ihrem inneren Ende mit der dort angeordneten Elektronik kontaktverbindbar sind, insbesondere mittels des Reflow-Lötens.

Diese Aufgabe wird laut Anspruch 1 dadurch gelöst, dass der Anschlussstift einen biokompatiblen Abschnitt und im Inneren des Implantates einen mit niedriger Energie fügbaren, vorzugsweise weichlötbaren Abschnitt aufweist, wobei der mit niedriger Energie fügbare Abschnitt des Anschlussstifts ein Aufsatz ist, der sich am inneren Ende des Anschlussstiftes befindet und vorzugsweise aus Nickel, Kupfer, Palladium, Gold, Silber, Eisen oder Legierungen aus diesen Materialien besteht, und wobei der Aufsatz im Inneren des Implantates als Stift ausgebildet ist.

Das innere Ende des Anschlussstifts und/oder des inneren Abschnittes kann zusätzlich nagelkopfförmig ausgebildet sein.

Der mit niedriger Energie fügbare, vorzugsweise weichlötbare, Abschnitt des Anschlussstiftes bringt den Vorteil mit sich, dass der Anschlussstift im Inneren des Implantates sieher und einfach mit niedriger Energie - beispielsweise niedriger Wärmeenergie bis 450°C, welche vor allem in Weichlötprozessen genutzt wird - fügbar ist. Insbesondere kann in einem Reflow-Prozess gleichzeitig eine kostengünstige Weichverlötung zusammen mit anderen Komponenten auf der Elektronikplatine des Implantats hergestellt werden. Dabei lassen sich diese Abschnitte der Anschlussstifte zusammen mit den anderen Komponenten der elektrischen Durchführung in Form der mindestens einen Durchführungshülse gleichzeitig in einem gemeinsamen Hochtemperatur-Lötprozess montieren, was wiederum eine besonders kostengünstige Anbindungsweise darstellt. Ferner wird durch Verwendung des mit niedriger Energie fügbaren Abschnitts des Anschlussstiftes im Inneren des Implantates die Ausbildung von Sprödphasen zwischen dem Abschnitt des Anschlussstiftes und dem verwendeten Weichlot vermieden, wie sie ansonsten beispielsweise zwischen Goldelektroden und Weichloten mit Zinn-Bestandteilen ausgebildet werden.

Optional können die mit niedriger Energie fügbaren Abschnitte des Anschlussstiftes zusätzlich noch mit besonders gut weich lötbaren, d. h. leicht benetzbaren Beschichtungen mit Materialien wie Palladium (Pd), Silber (Ag), Kupfer (Cu), Gold (Au) sowie Legierungen aus diesen Materialien versehen sein, wobei die Beschichtung eine Schichtdicke bis zu etwa 0,5 mm und im Falle einer Goldbeschichtung eine Dicke bis 200 µm aufweist. Eine Goldbeschichtung mit dieser Schichtdicke bildet zusammen mit Weichlotmaterialien enthaltend Zinn-Bestandteile bekanntermaßen keine Sprödphasen aus.

Zusammenfassend werden aufgrund der erfindungsgemäßen Ausgestaltung der elektrischen Durchführung zur Implantat-Außenseite hin nur biokompatible Oberflächen und nach innen hin weich lötbare Elektrodenbereiche angeboten. Letztere sind beispielsweise dafür geeignet, in einem Reflow-Lötverfahren zur Kontaktierung weiter verarbeitet zu werden. Materialien für den biokompatiblen Abschnitt des Anschlussstifts sind Nb, Ta, Ti, Pt, Pt/Ir, Zr, Hf, medizinische Edelstähle wie 316L oder Legierungen aus diesen Materialien, sowie FeNi, FeNiCo, FeCr, Mo, W, Cr, V, Al oder Legierungen aus diesen Materialien. Materialien für den mit niedriger Energie fügbare Abschnitt sind Nickel, Kupfer, Palladium, Gold, Silber, Eisen oder Legierungen aus diesen Materialien. Diese Legierungen können zusätzlich zu den genannten Elementen noch ein oder mehrere der folgenden Elemente enthalten: Zink (Zn), Zinn (SN), Cadmium (Cd), Blei (Pb), Antimon (Sb), Arsen (As), Wismut /Bismut (Bi), Phosphor (P), Silizium (Si), Stickstoff (N) oder Beryllium (Be).

Der mit niedriger Energie fügbare Abschnitt aus den genannten Materialien ist dabei ein Aufsatz, der sich auf dem inneren Ende Anschlussstift befindet. Dieser Aufsatz kann einerseits als Stift ausgebildet sein, der vorteilhaft sich in Verlängerung der Längsachse des Anschlussstiftes befindet und durch fehlende Verdickungen die problemlose Aufnahme weiterer Komponenten wie Filter zur Sicherstellung der elektromagnetischen Verträglichkeit (EMV-Filter) in Form von Kondensatoren ermöglichen. Alternativ kann der Aufsatz auch als Scheibe bzw. Ronde ausgebildet sein, die als Vorteil beim Reflow-Prozess den korrespondierenden Kontaktstellen auf der Partner-Platine größere Flächen bietet und mechanisch festere und belastbarere Verbindungen ermöglicht.

Vorzugsweise ist der mit niedriger Energie fügbare Aufsatz mittels einer Fügestelle an den biokompatiblen Abschnitt des Anschlussstiftes angebunden, insbesondere Hartlote Legierungen enthaltend Kupfer (Cu), Silber (Ag), Kupfer-Nickel (CuNi), Kupfer-Zink (CuZn), Kupfer-Zinn (CuSn), Silber-Kupfer (AgCu), Silber-Kupfer-Zink (AgCuZn), Silber-Kupfer-Zink-Zinn (AgCuZnSn), Silber-Kupfer-Zinn (AgCuSn), Silber-Kupfer-Zink-Kadmium (AgCuZnCd), Kupfer-Phosphor (CuP), Kupfer-Phosphor-Silber (CuPAg), Kupfer-Gold (CuAu), mit welchen Temperatur-Inhomogenitäten während des Hartlötprozesses ausgeglichen werden können. Bevorzugt wird die harte Anlötung mit Goldlot/Goldlotlegierungen. Möglich sind auch weitere Legierungen daraus mit zusätzlich möglichen Legierungszuschlägen wie Pb, Sb, As, Bi, P, N, Be, Ni. Weiter kann der mit niedriger Energie fügbare Aufsatz mittels einer Fügestelle an den biokompatiblen Abschnitt des Anschlussstiftes angelötet, angeschweißt, angecrimpt, angeklemmt oder elektrisch leitfähig angeklebt, aber vorzugsweise mit Goldlot hart angelötet. Vorzugsweise befindet sich die Fügestelle bezüglich des Verbindungslotes innerhalb des Implantatsgehäuses. Dies ist die bevorzugte Anbindungsart, da diese einfach, wegen fehlender Sprödphasen zuverlässig, mechanisch belastbar und gleichzeitig zusammen mit den weiteren Durchführungskomponenten im selben Lötprozess der Durchführung realisierbar ist. Da diese Anbindung bereits vor der Anbindung mit der elektrischen Schaltung im Inneren des Implantats erfolgt, kann dort an der Fügestelle ein Schweiß- oder Hartlötvorgang erfolgen. In einer bevorzugten Ausführung kann sich die Fügestelle innerhalb der mindestens einen Durchführungshülse befinden. Dadurch lässt sie sich aufgrund der zentrierenden Wirkung der Durchführungshülse im Lötprozess der Durchführung einfach realisieren und zusätzlich vor mechanischen Belastungen und weiteren Einflüssen schützen. Weiterhin ist es besonders bevorzugt möglich, diese auch im Glaslot einzuschließen. Dies führt zu einem weitergehenden Schutz vor mechanischen Belastungen, da die Fügestelle und die angrenzenden Stift-Bereiche durch das Glaslot nach außen hin mechanisch entkoppelt sind.

In einer weiteren bevorzugten Ausführung umfasst die elektrische Durchführung eine äußere und eine innere Durchführungshülse. In dieser Ausführung ist der mindestens eine Anschlussstift mit der äußeren und der inneren Durchführungshülse und die äußere und innere Durchführungshülse mit dem Flansch mittels einer als Glaspfropf ausgebildeten Lotverbindung hermetisch dicht verbunden. Der Glaspfropf wird durch einen Hohlraum begrenzt, der vom Flansch, von der äußeren und von der inneren Durchführungshülse umschlossen ist. Als Lotmaterial wird bevorzugt das eingangs erwähnte Glaslot vom Typ 8625 der Firma Schott verwendet. Dadurch werden die Durchführungshülsen während der Verlötung als Fließbarriere genutzt, was zu einer Vereinfachung und einer Ausbeute-Steigerung des Herstellprozesses führt. In weiteren Ausführungsformen können entweder die äußere oder die innere Durchführungshülse oder auch beide entfallen, wenn dafür geeignete Materialien bzw. Materialkombinationen für den Flansch, die Anschlussstifte und das Glaslot gewählt werden. Diese Varianten haben den Vorteil, dass die Verlötungen insgesamt Raum sparender als mit gleichzeitig zwei Durchführungshülsen ausgeführt werden können.

Durch moderne Bildgebungsverfahren und auch wegen der in der Umgebung vorhandenen Strahlung beispielsweise durch Mobiltelefone, Funknetze, Magnetresonanztomographen und dergleichen ist die Sicherheit gegen Einstrahlung in das Gehäuse und damit die Verhinderung von Strahlungseintrag sehr wichtig. Aus diesem Grunde kann die elektrische Durchführung ein Filter, vorzugsweise einen Filterkondensator, umfassen, der am mit niedriger Energie fügbaren Abschnitt, vorzugsweise am weichlötbaren Stift, elektrisch verbunden ist. Durch das Filter wird eine Schirmung zwischen dem Flansch und dem mindestens einen Anschlusspin hergestellt und dadurch die innerhalb des Gehäuses liegende Schaltung und die weiteren Komponenten gegen elektromagnetische Einstrahlung geschützt. Die geeigneten Filter sind meist Keramiken, die im Allgemeinen sehr wärme- und bruchempfindlich sind. Somit können diese direkt ohne weitere aufwändige Maßnahmen nur mit einem niedrig-energetischen Verfahren wie einem Weichlötverfahren angefügt werden.

Die Erfindung umfasst weiterhin ein Herstellverfahren für eine elektrische Durchführung, bei dem während der Erzeugung des Glaslot-Pfropfs der Anschlussstift mittels einer Wärmesenke gekühlt wird. Dadurch, dass hierbei der Anschlussstift kühler bleibt als das Glas, lassen sich auch bei Verwendung von Anschlussstift-Materialien mit ansonsten gegenüber dem Glaslot inkompatiblen Wärmeausdehnungskoeffizienten die auftretenden thermischen Spannungen so weit kontrollieren, dass eine hermetisch dichte und mechanisch belastbare Verlötung gelingt. Der Wärmeeintrag in das Glaslot kann beispielsweise durch IR-Strahlung (etwa eines CO₂-Lasers) oder induktive Wärmeeinkopplung über den umgebenden Flansch o. ä. erfolgen.

Weiterhin umfasst die Erfindung die Verwendung der erfindungsgemäßen Durchführung, ein Weichlöten am Anschlussstift mittels eines Reflow-Verfahrens ausgeführt wird. Zusätzlich kann gleichzeitig mit dem innenseitigen Weichlöten ein außenseitiges Weichlöten mittels eines Reflow-Verfahrens ausgeführt werden.

In den abhängigen Ansprüchen sind bevorzugte Weiterbildungen der Anschlusselektroden-Durchführung angegeben, deren Merkmale, Einzelheiten und Vorteile aus der folgenden Beschreibung des Ausführungsbeispiels anhand der beigefügten Zeichnung deutlich werden.
- Fig. 1: zeigt eine räumliche Darstellung einer erfindungsgemäßen Durchführung mit zehn Anschlussstiften für Implantat-Signale mit einem elften Anschlussstift für die elektrische Masseanbindung.
- Fig. 2: zeigt eine Schnittdarstellung einer weiteren Ausführungsform der erfindungsgemäßen Durchführung.
- Fig. 3: zeigt eine Schnittdarstellung einer Variante der erfindungsgemäßen Durchführung aus Fig. 2 mit vier nagelkopfförmigen Anschlussstiften für Implantat-Signale und einem nagelkopfförmigen Massestift
- Fig. 4: zeigt eine Schnittdarstellung einer weiteren Variante der erfindungsgemäßen Durchführung mit fünf Anschlussstiften, die mit einer das Reflow-Weichlöten verbessernden Beschichtung (Vorverzinnung) versehen sind
- Fig. 5: zeigt eine Schnittdarstellung der zusätzlichen Variante der erfindungsgemäßen Durchführung aus Fig. 2 mit fünf Anschlussstiften, wobei eine Durchführungshülse mindestens zwei Anschlusselektroden führt und die Anschlussstifte mit einer die Weichlötbarkeit verbessernden Beschichtung versehen sind.
- Fig. 6: zeigt eine Schnittdarstellung einer alternativen Ausführungsform der erfindungsgemäßen Durchführung mit fünf Anschlusselektroden und mit Glas verlöteten Komponenten
- Fig. 7: zeigt eine Schnittdarstellung einer weiteren Ausführungsform der erfindungsgemäßen Durchführung mit einem Filterkondensator

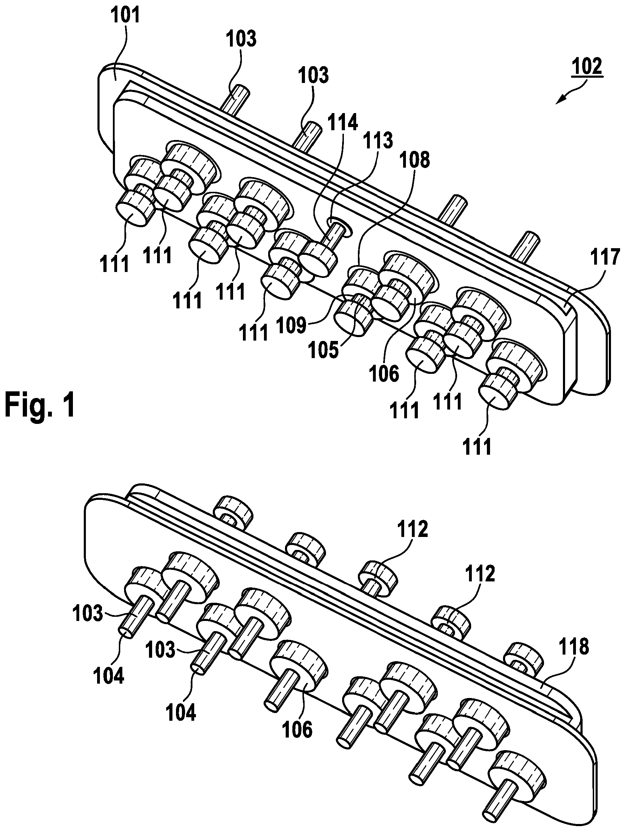

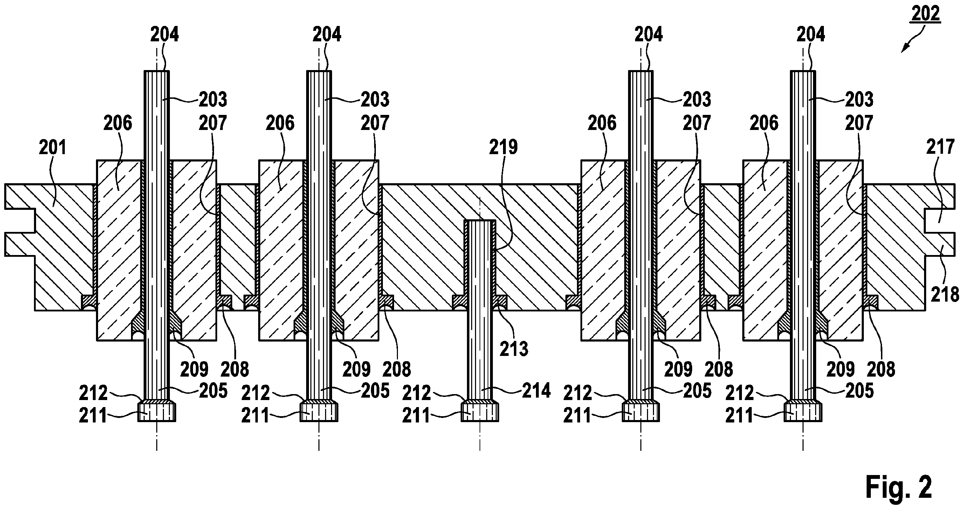

Fig. 1 zeigt allgemein eine elektrische Durchführung in räumlicher Darstellung eines grundsätzlichen Aufbaus einer Reihendurchführung 102 mit einer Ansicht der Innenseite (in das Implantatsinnere ragend) und der Außenseite. Die elektrische Durchführung umfasst einen Flansch 101, der vorzugsweise aus Ti, Nb, Ta, Zr, Legierungen aus einem oder mehreren der Elemente oder weiterer Zusatz-Elemente wie Hf, Al, Fe, P, Si, Mn oder C, oder aus Keramik besteht, in dem sich mehrere Durchführungshülsen 106 befinden, mit welchen Anschlussstifte 103 durch Lotverbindungen verbunden sind. Auf der Innenansicht ist ein weichlötbarer Aufsatz 111 des Anschlussstiftes 103 in Form einer Scheibe zu erkennen, welcher durch eine Fügestelle 112 elektrisch und mechanisch an den Anschlussstift 103 angefügt ist. Die dort bezeichneten Komponenten werden weiter in Fig. 2 beschrieben. Die Durchführung ist in dieser und den folgenden Fig. als Reihendurchführung mit einer Reihe aus zehn Durchführungshülsen 106 und Anschlussstiften 103 und Reihen mit vier bzw. sechs solcher Durchführungshülsen und Anschlussstiften aufgebaut. In Letzterer befindet sich zusätzlich ein Massestift 114, welcher ebenfalls einen weichlötbaren Aufsatz 11 aufweist.

Fig. 2 zeigt den Schnitt einer weiteren Ausführungsform der elektrischen Durchführung 202. Gleiche oder ähnliche Bauteile sind mit an Fig. 1 angelehnte Bezugsziffern bezeichnet und werden hier nicht nochmals erläutert. Beispielsweise bezeichnet die Bezugsziffer 102 in Fig. 1 die gleiche Komponente wie die Bezugsziffer 202 in Fig. 2.

Der Flansch 201 führt vorzugsweise mindestens eine zylindrische Durchführungshülse 206 aus biokompatiblem Al₂O₃ in seinen Flanschöffnungen 207. Jede der Hülsen ist mit einer Lotverbindung 208 aus biokompatiblem, metallischem Hartlot mit dem Flansch 201 verlötet. Die Durchführungshülsen 206 sind in dieser Ausführungsform im Bereich der Flanschöffnungen 207 mit einer metallischen Beschichtung versehen, vorzugsweise biokompatibel aus Nb, um diese für das Hartlot benetzungsfähig zu machen und dadurch eine Verlötung zu ermöglichen. In der hier gezeigten Variante überragen die keramischen Durchführungshülsen 206 den Flansch 201 beidseitig und sorgen für ausreichend lange elektrische Isolationsstrecken für Hochspannungsanwendungen der Durchführung 202.

Der Anschlussstift 203 hat vorzugsweise eine einfache zylindrische Form, ein äußeres Ende 204 und ein inneres Ende 205, und ist mittels einer Lotverbindung 209 mit der Durchführungshülse 206 verbunden und damit zusammen mit der Durchführungshülse im Flansch 201 festgelegt. Sowohl die Lotverbindung 208 als auch 209 sind in der Produktionsphase als Lotringe ausgebildet, die sich zwischen dem Flansch 201 und den Durchführungshülsen 206 bzw. zwischen Durchführungshülsen 206 und Anschlussstiften 203 befinden. Durch Erhitzen, beispielsweise über elektrische Widerstandsbeheizung, elektrische Induktion, Wärmeleitung oder Infrarot-Einstrahlung, werden diese Ringe verflüssigt und bilden nach Erkalten eine biokompatible, mechanisch stabile, hermetisch dichte, Wechseltemperatur belastbare Lotverbindung.

Am inneren Ende 205 ist ein weichlötbarer Aufsatz 211, vorzugsweise in Form von Nickel-Scheiben über eine Fügestelle 212 angefügt. Um die genannten Lotverbindungen 208, 209 und Fügestellen 212 im selben Prozess kostengünstig herstellen zu können, bestehen die Materialien der Lotverbindungen und der Fügestelle vorzugsweise aus demselben Lotmaterial wie beispielsweise Goldlote, Gold-Niob-, Gold-Tantal, Gold-Titan, Gold-Zirkonium-Legierungen. Alternativ können z. B. auch Kupfer-, Silber-, Kupfer-Nickel-, Kupfer-Zink-, Kupfer-Zinn, Silber-Kupfer-, Silber-Kupfer-Zink-, Silber-Kupfer-Zink-Zinn-, Silber-Kupfer-Zinn-, Silber-Kupfer-Zink-Kadmium-, Kupfer-Phosphor-, Kupfer-Phosphor-Silber-, Kupfer-Gold-Legierungen oder zahlreiche weitere Legierungen eingesetzt werden, um Temperatur-Inhomogenitäten während des Hartlötprozesses auszugleichen. Diese Implantat-innenseitig liegenden Fügestellen müssen wie auch die Implantat-innenseitig liegenden Anschlussstifte nicht biokompatibel ausgeführt sein, da sie durch das hermetisch dichte Implantatgehäuse und die hermetisch dichte Durchführung zur Außenseite hin getrennt sind.

Als bevorzugte Materialkombination wird Niob für den Anschlussstift 203 bzw. 214, Nickel für den weichlötbaren Aufsatz 211 und Feingold für das Hartlot 212 bzw. 215 gewählt, da Feingold sowohl mit Niob als auch mit Nickel Lotverbindungen erzeugt, die in jedem Legierungsverhältnis miteinander mischbar und stets duktile Phasen bilden. Die resultierenden Hartlotverbindungen sind derart stabil, dass bei mechanischer Belastung in den meisten Fällen die Anschlussstifte 203 bzw. 214 reißen bzw. versagen und nicht die Hartlotverbindungen 212.

Alternativ ist es prozesstechnisch aber auch vorteilhaft, in einem ersten Verfahrensschritt die Fügestelle 212 zwischen Anschlussstift 203 und Aufsatz 211 mit einem höher schmelzenden Hartlot zu fügen oder ohne Zusatz direkt zu verschweißen, um diese anschließend in einem zweiten Hartlötprozess mit den anderen Komponenten der Durchführung 202 zu verlöten, so dass mögliche Probleme - beispielsweise bei komplexen und/oder großräumigeren Strukturen - mit unverwünschten Temperatur-Inhomogenitäten vermieden werden. Wesentlich ist, dass kein Weichlot mit Zinnanteil in der Fügestelle 212 eingesetzt wird, um mechanisch wenig belastbare Sprödphasen zwischen Gold und Zinn zu vermeiden.

Weiter umfasst die elektrische Durchführung gemäß der gezeigten Ausführungsform einen Massestift 214, für den sie soeben dargestellten Betrachtungen bezüglich der Lotverbindungen ebenfalls gelten.

Die inneren Flächen der Aufsätze 211 des Anschlussstifte 203 und des Massestiftes 214 befinden sich vorzugsweise alle annähernd in einer gemeinsamen Ebene und ermöglichen damit eine erfolgreiche Reflow-Verlötung, können aber auch in gewollt unterschiedlichen Ebenen liegen, wenn es die Anpassung an das korrespondierende Substrat des Implantats erfordert.

In der hier gezeigten Ausführung weist der Flansch 201 eine Nut 217 zur Aufnahme der Halbschalen eines nicht dargestellten Implantatgehäuses auf. Eine Lippe 218 dient gleichzeitig als Schweißschutz beim Laser-Einschweißen des Flansches 201 mit den Gehäusehalbschalen des Implantats.

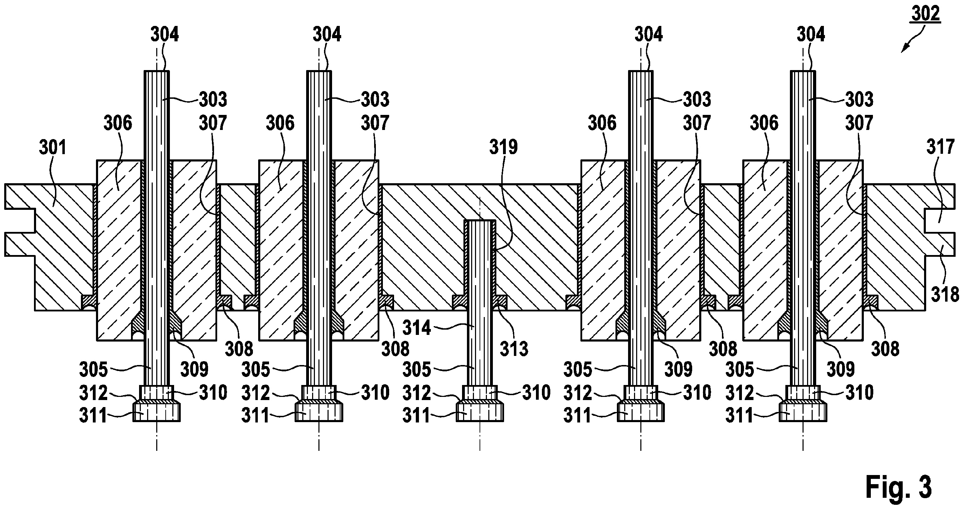

Fig. 3 zeigt eine weitere Variante zu den Ausführungsformen aus Fig. 1 und Fig. 2. Gleiche oder ähnliche Bauteile sind mit an Fig. 2 angelehnten Bezugsziffern bezeichnet und werden hier nicht nochmals erläutert. Die Stifte 304 weisen gemäß der gezeigten Ausführungsform am inneren Ende 305 nagelkopf- oder tellerartige Ansätze (auch "Nailheads" genannt) 310 auf. Dadurch wird eine bessere Ausrichtung der weichlötbaren Aufsätze 311 erleichtert und eine genauere gemeinsame Ebenheit erzielt.

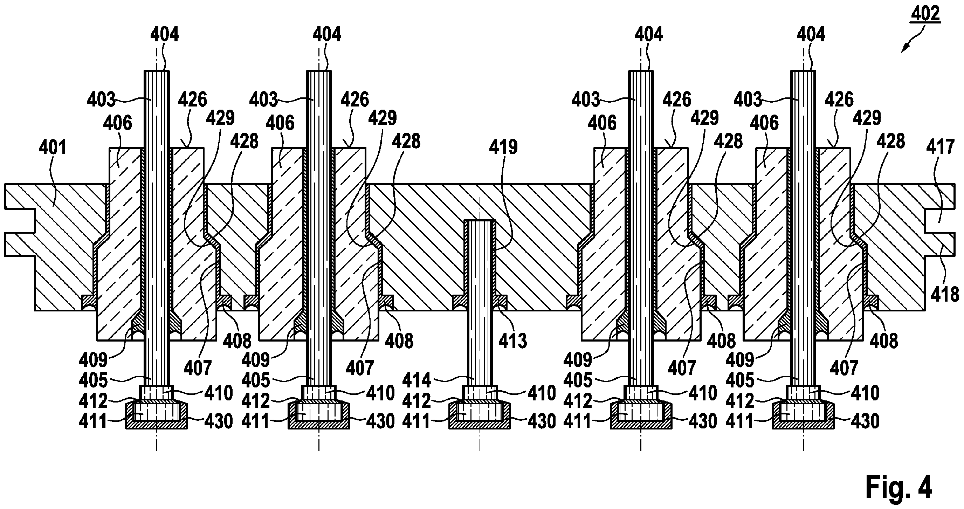

Fig. 4 zeigt eine weitere oder zusätzliche Variante zu den vorherig dargestellten Ausführungsformen. Gleiche oder ähnliche Bauteile sind mit an die vorher beschriebenen Figuren angelehnten Bezugsziffern bezeichnet und werden hier nicht nochmals erläutert. Vor dem eigentlichen Reflow-Prozess sind die weichlötbaren Aufsätze 411 der Anschlussstifte 403 und der Massestifte 414 mindestens an ihrer Stirnfläche mit einer Beschichtung oder Schicht 430, vorzugsweise aus Weichlot Sn37Pb z. B. von den Firmen Weidinger oder Zevaton benetzt, wobei für eine gute Benetzung der Stirnflächen der Aufsätze 411 geeignete Flussmittel als Hilfsmittel verwendet werden, vorzugsweise vom genormten Typ "Alpha 850-33".Aber auch andere Flussmittel wie beispielsweise wässrige oder Salzsäurehaltige Lösungen aus Zinkchlorid/Ammoniumchlorid, alkoholbasierte Lösungen mit Dimethylaminhydrochlorid, oder wässrige Lösungen aus stark aktivierten Halogeniden sind geeignet. Alle diese Flussmittel bergen den Vorteil, dass sie nach der erfolgten Benetzung der Aufsätze 411 mit Weichlot 430 in einem einfachen Reinigungsverfahren mit wässrigen Lösungen ohne Rückstände von der Durchführung wieder entfernt und mögliche Leckpfade in der Durchführung nicht durch Flussmittelrückstände verdeckt werden, so dass die Durchführungen auf sichere Art und Weise vorzugsweise mit Helium-Lecktests auf Hermetizität geprüft werden können.

Die Weichlotoberfläche ist zusätzlich durch ein besonderes Verfahren - beispielsweise durch thermische Vorbehandlung oder durch Schleifen - in eine gemeinsame Ebene gebracht. Mit den Schichten 430 aus Weichlot wird im Allgemeinen eine bessere gemeinsame Ebenheit erreicht als es mit den Aufsätzen 411 allein möglich ist, sodass mit der Schicht produktionsbedingte Unebenheiten der weichlötbaren Aufsätze 411 ausgeglichen werden. Weiterhin schafft die Beschichtung günstige Voraussetzungen für ein Reflow-Weichlötverfahren, da es nicht mehr notwendig ist, erst während des Reflow-Lötens eine möglichst vollständige Benetzung der Aufsätze 411 mit Weichlot zu erzielen, da die Oberflächen der Aufsätze 411 bereits nahezu vollständig mit Weichlot benetzt sind. Die Seitenflächen der scheibenförmigen Aufsätze 411 können ebenfalls durch die Weichlotschicht 430 benetzt sein. Selbst wenn die goldlothaltigen Fügestellen 412 unabsichtlich mit zinnhaltigem Weichlot mit benetzt werden und dabei in den Übergangszonen Au-Sn-Sprödphasen ausgebildet werden, stellen die Au-Sn-Sprödphasen in dieser Anordnung keinen Nachteil dar, da die Au-Sn-Sprödphasen mechanisch keine Funktion übernehmen, mechanisch nicht nennenswert belastet werden und mit dem Rest der Weichlotschicht 430 duktil zusammenhängend sind, so dass sich keine Bestandteile oder Partikel des Weichlotes 430 in der weiteren Anwendung der Durchführung ablösen. Die inneren Enden 405 des Anschlussstiftes 403 oder des Massestiftes 414 können auch, wie in Fig. 3 dargestellt, nagelkopfförmig sein.

Verfahrenstechnisch vorteilhaft weisen sowohl die Durchführungshülsen 406 als auch die Innenwände der Öffnungen 407 des Flansches 401 korrespondierende Schrägen oder Stufen 428 und 429 auf. Damit wird eine verjüngte Hülsenaußenfläche 426 gebildet, die im Allgemeinen weiter aus dem Flansch 401 herausragt als ohne Verjüngung, um eine ausreichende Isolationsstrecke zu gewährleisten. Mit dieser Konfiguration kann die Keramik vor dem Erstellen der Lötverbindung 408 im inneren Hohlraum des Flansches zentriert und muss nicht durch zusätzliche Hilfsmittel in Position gehalten werden.

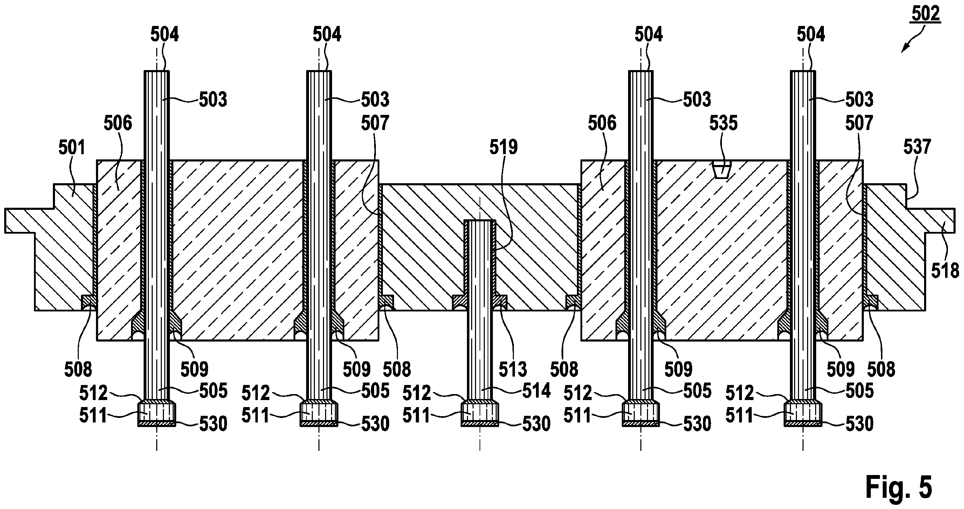

Fig. 5 zeigt eine weitere, zu den bisherigen Ausführungsformen kompatible Variante, in der zwei oder mehrere Stifte 503 in einer gemeinsamen Keramik 506 eingelötet sein können. Gleiche oder ähnliche Bauteile sind mit an die vorher beschriebenen Figuren angelehnten Bezugsziffern bezeichnet und werden hier nicht nochmals erläutert. Die gemeinsamen Keramiken 506 können Vertiefungen oder sog. "Slots" 535 besitzen, die die elektrische Isolationsstrecken zwischen den Stiften 503 untereinander und/oder den Stiften 503 und dem Flansch 501 verlängern und dadurch die Hochspannungsfestigkeit der Durchführung erhöhen. In dieser Ausführungsform sind die weichlötbaren Aufsätze 511 mit einer besonders gut weichlötbaren Beschichtung 530 versehen, beispielsweise aus Palladium, Silber, Gold, Kupfer oder Legierungen aus diesen Materialien. Besonders bevorzugt sind Gold-Beschichtungen mit Dicken von bis zu etwa 200 µm, da diese bei diesen Schichtstärken keine Sprödphasen mit dem Zinn des Weichlotes ausbilden. Die beschichteten Aufsätze 511werden vorzugsweise aus beidseitig beschichteten Ni-Blechen oder -Folien ausgestanzt und sind deshalb vorzugsweise nur an einer Stirnfläche mit besonders gut weichlötbaren Beschichtungen 530 für den anschließenden Reflow-Prozess versehen. Die Beschichtung 530 und die zum Stift hin weisende andere Beschichtung, an der sich die Fügestelle 512 können aus unterschiedlichen Materialien bestehen und unterschiedliche Dicken besitzen, wobei sich die zum Stift hin weisende andere Beschichtung besonders für das Hartlöten mit den Anschlussstiften 503 und dem Massestift 514 eignet und die andere Beschichtung 530 für das Reflow-Weichlöten. Die Aufsätze 511 können auch an ihren Mantelflächen zusätzlich besonders gut weichlötbare Beschichtungen besitzen, die in diesem Falle für eine verbesserte mechanische Belastbarkeit der beim Reflow-Weichlöten hergestellten Weichlötverbindungen sorgen.

In dieser Variante besitzt der Flansch 501 eine Einpassung 537, welche zum Einschweißen in eine Öffnung des nicht dargestellten Implantatgehäuses dient. Ein Anschlag bzw. eine Lippe 518 dient gleichzeitig als Schweißschutz beim Laser-Einschweißen des Flansches 501 mit dem Gehäuse bzw. den Gehäusehalbschalen des Implantats.

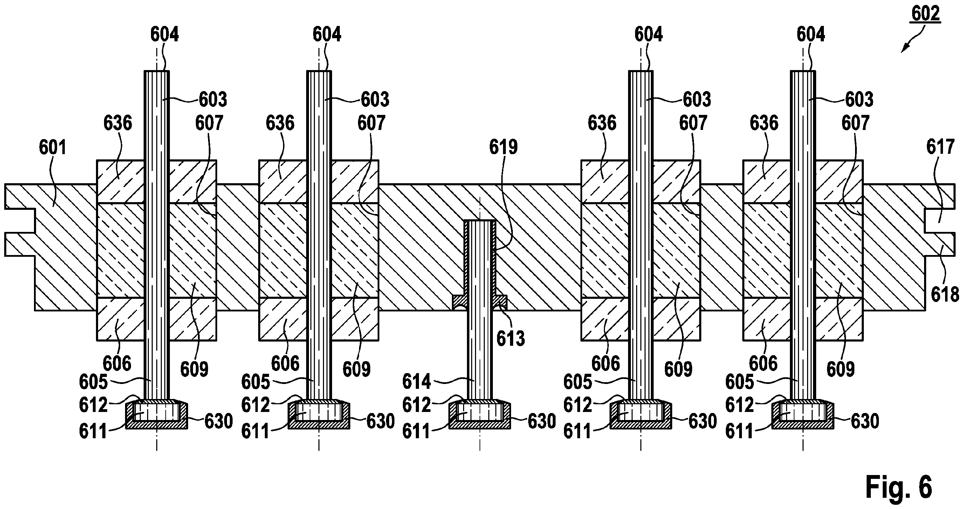

Eine besondere Ausführungsform ist in Fig. 6 dargestellt. Gleiche oder ähnliche Bauteile sind mit an die vorher beschriebenen Figuren angelehnten Bezugsziffern bezeichnet und werden hier nicht nochmals erläutert. In jeder inneren Öffnung 607 des Flansches 601 sind eine innere Durchführungshülse 606 und eine äußere Durchführungshülse 636 angebracht, welche einen Hohlraum mit dem Flansch 601 bilden. Die Anschlussstifte 603 sind mittels eines vorzugsweise biokompatiblen Glaslotes 609 mit dem Flansch 601 und den Durchführungshülsen 606 und 636 verlötet, wobei sich das Glaslot im Hohlraum befindet und diesen komplett oder nahezu komplett ausfüllt. Die Durchführungshülsen 606 und 636 bilden für das Glaslot 609 während der Verlötung eine Fließbarriere, d. h. sie verhindern während des Lötprozesses ein Wegfließen des Glaslotes 609 aus den Öffnungen 607 des Flansches 601. Bei Wahl des richtigen Glaslotes 609, vorzugsweise Glaslot des Typs 8625 der Firma Schott, kann im selben Löt- bzw. Erhitzungsprozess sowohl der Massestift 614 mittels Hartlot 613 in der Öffnung 619 des Flansches 601 verlötet werden als auch die Aufsätze 611 mittels der Fügestellen 612, da Glaslot im Allgemeinen einen weiten Temperaturbereich der Verarbeitung ermöglicht. Im Falle einer Glas-/Keramik-Durchführung besteht die Lotverbindung 609 vorzugsweise aus einem biokompatiblen Glaslot, das den Flansch 601, die Anschlussstifte 603 und die Durchführungshülsen 606 und 636 gleichzeitig benetzt und dessen Wärmeausdehnungskoeffizient an die benetzten Komponenten vorzugsweise angepasst ist.

Gegebenenfalls können in weiteren Varianten die Durchführungshülsen 606 und/oder 636 gleichzeitig weggelassen werden, wenn dafür geeignete Materialien bzw. Materialkombinationen für den Flansch, die Anschlussstifte und das Glaslot gewählt werden. In solchen Fällen ist Beispielsweise das Glaslot von seiner Zusammensetzung her weniger oxidierend oder sogar reduzierend eingestellt, so dass die Metalloberflächen für das Glaslot weniger attraktiv wirken, die Oberflächenspannung des Glaslotes in der Hartverlötung dominiert und letztendlich das Glas trotz niedriger Viskosität während der Verarbeitung nicht aus den Öffnungen 607 heraus fließt. Diese Varianten haben den Vorteil, dass die Verlötungen insgesamt Raum sparender als mit gleichzeitig zwei Durchführungshülsen ausgeführt werden können. Die Öffnungen 607 können in weiteren Varianten Schrägen oder Stufen wie in Fig. 4 beschrieben, besitzen, die für das Glaslot eine Positionierhilfe in den Öffnungen 607 bilden.

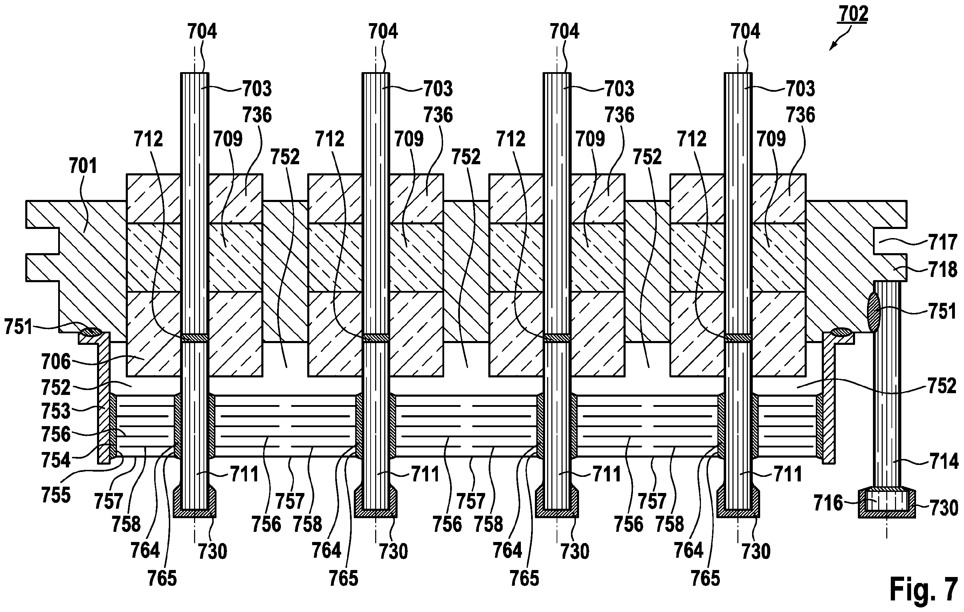

Fig 7 zeigt eine weitere Ausführungsform der Erfindung. Gleiche oder ähnliche Bauteile sind mit an die vorher beschriebenen Figuren angelehnten Bezugsziffern bezeichnet und werden hier nicht nochmals erläutert. In Fig. 7 ist eine gefilterte Durchführung 702 mit einem Flansch 701 dargestellt, welche eine innere Durchführungshülse 706, eine äußere Durchführungshülse 736 und ein dazwischen liegender Glaslotpfropf 709 aufweist. In jeder dieser Durchführungshülsen 706 und 736 ist ein durch den Glaslotpfropf 709 festgelegter Anschlussstift dargestellt, der zweiteilig ausgeführt ist. Er besteht aus einem äußeren, biokompatiblen Abschnitt 703, an dem an einer Fügestelle 712 ein weichlötbarer Stift 711 angefügt ist. Das Lotmaterial der Fügestelle 712 kann eines der vorher genannten Lotmaterialien sein. Die Fügestelle 712 kann dabei während des Herstellverfahrens durch den Glaslotpfropf so festgelegt worden sein, dass sie sich innerhalb der inneren Durchführungshülse 706 befindet, um so gegen mechanische und chemische Einflüsse geschützt zu sein. Weiter umfasst die elektrische Durchführung einen Massestift 714, der in dieser Ausführungsvariante mittels einer Schweißstelle 751 elektrisch und mechanisch mit dem Flansch 701 verbunden ist. Der Massestift kann aus demselben biokompatiblen Material wie der biokompatible Abschnitt 703 des Anschlussstiftes bestehen, aber auch aus dem gleichen Material, aus dem der Flansch 701 besteht, was die Schweißbarkeit verbessert, umfasst jedoch einen weichlötbaren Abschnitt 716 in Form einer Scheibe. Sowohl die weichlötbaren Stifte 711 als auch der weichlötbare Abschnitt 714 des Massestifts können zur besseren Anbindung mit einer Weichlotbeschichtung 730 versehen sein und durch ein besonderes thermisches Verfahren oder durch Schleifen eine nahezu gemeinsame Ebene (engl. "common zone") für den Reflow-Prozess bilden.

Zur elektromagnetischen Filterung wird in dieser Variante ein Filterkondensator 757 eingesetzt, der mittels einer Hülse 753 am Flansch gehalten wird. Die Hülse 753 ist mittels einem oder mehrerer Schweißpunkte oder -Nähte 751 am Flansch 701 elektrisch und mechanisch angefügt, wobei der Flansch 701 zur besseren Positionierung der Hülse 753 bezüglich des Flansches 701 eine Vertiefung aufweisen kann. Die Schweißpunkte 751 sind dabei so angelegt, dass zwischen der Hülse 753 und dem Flansch 701 eine Lecktestverbindung geschaffen wird.

Der Filterkondensator 757 umfasst plattenförmige Elektroden 756 und 758, die in einem Dielektrikum 757, welches beispielsweise aus Bariumtitanat besteht, eingebettet sind. Die Elektroden 756 weisen an der Außenseite des Kondensators eine weichlötbare Metallisierung 755 aus beispielsweise Palladium, Silber, Kupfer oder deren Legierungen auf. An dieser Metallisierung erfolgt eine elektrische und mechanische Masseverbindung über eine feste Lötverbindung 754 mit der am Flansch 701 befestigten Hülse 753. Die Elektroden 758 sind ebenfalls mit einer weichlötbaren Metallisierung 765 ebenfalls aus Palladium, Silber, Kupfer oder deren Legierungen an den Öffnungen des Kondensators versehen, durch die sich die weichlötbaren Stifte 711 erstrecken und mittels der Lötverbindung 764 mit den weichlötbaren Stiften 711 verlötet sind, um die elektrischen Verbindungen zu den elektrischen Signalen des elektrischen Implantats zu bilden. Die Lötverbindungen 764 und 754 können aus Gründen der besseren Fertigung aus verschiedenartigen Weichloten mit unterschiedlichen Schmelzpunkten bzw. -bereichen bestehen. Dabei wird für als Material für die Lötverbindung 754 vorzugsweise eine Materialzusammensetzung gewählt, beispielsweise PbSn3,5Ag1,5, die mit einem Lötbereich von 305°C eine höhere Verarbeitungstemperatur besitzt als das Material der Lötverbindung 764 mit der bevorzugten Materialzusammensetzung von beispielsweise PbSn5Ag2,5 und einer Löttemperatur von 280°C. Somit kann in einem bevorzugten Herstellungsverfahren der Kondensator 757 zunächst an die weichlötbare Hülse 753 mit dem höher schmelzenden Weichlot 754 angelötet werden, die Hülse 753 mit dem angelöteten Kondensator 757 über die Stifte 711 gesteckt und an den Flansch 701 mittels Verschweißungen 750 elektrisch und mechanisch angefügt werden und schließlich mittels eines niedriger schmelzenden Weichlotes 764 an die weichlötbaren Stifte 711 angelötet werden, ohne dass dabei das höher schmelzende Weichlot 754 Gefahr läuft, während des zweiten Weichlötens mit dem Weichlot 764 erneut aufzuschmelzen und die Metallisierung 755 aufzulösen und dabei die elektrisch/mechanische Verbindung zu den Kondensatorelektroden 756 zu verlieren. Die so hergestellte elektrische Durchführung kann mittels eines Helium-Lecktests auf Hermetizität zwischen der Innen-und Außenseite des Implantats geprüft werden, da die Lecktestverbindung einen Durchgang zum von Flansch 701, Hülse 753, Kondensator 757 und den einzelnen inneren Durchführungshülsen 706 und weichlötbaren Stiften 711 begrenzen Hohlraum 752 schafft, durch den das Helium strömen kann.

In weiteren Varianten dieser Ausführungsform können anstatt eines einzelnen Kondensators 757 auch mehrere unabhängige Kondensatoren verwendet werden. Es ist auch möglich, nur einzelne Anschlussstifte mittels Kondensatoren 757 zu filtern (beispielsweise für eine Antennenanbindung zur drahtlosen Übertragung von Signalen aus dem Implantat heraus). Die elektrische und mechanische Anbindung des Kondensators 757 kann auch mittels elektrisch leitfähigen Klebern, mittels Schweißen, Klemmen oder Stecken realisiert sein, wobei stets auf die Lecktestbarkeit der Anordnung ein besonderer Wert gelegt wird. Die Lecktestbarkeit kann alternativ oder zusätzlich durch nicht dargestellte, zusätzliche Öffnungen im Kondensator 757, in den Loten 754 und 764 und/oder in der Hülse 753 realisiert sein. Der Flansch 701 kann alternativ so geformt sein, dass sich anstatt der Hülse 753 der Flansch an ähnlicher Stelle fortsetzt und den Kondensator 757 aufnimmt und zur Lecktestbarkeit eine zusätzliche Öffnung in der so entstandenen, hier nicht dargestellten Wandung zum Hohlraum 752 besitzt.

In allen genannten Ausführungsformen können auch andere als die genannten Fügetechniken und Ausformungen angewendet werden, wie beispielsweise durch Schweißen, Klemmen, elektrisch leitfähiges Kleben, Bonden und dergleichen.

In weiteren Varianten können die Stifte 711 auch mit den in den Fig. 2 bis 5 dargebotenen Beispielen mit dem Flansch 701 angebunden und gefiltert sein.

In weiteren sinnvollen Varianten können sämtliche Kombinationen und geometrische Modifikationen aus den Figuren 1 bis 7 realisiert werden und sind Bestandteil dieser Patentanmeldung.

### Bezugszeichenliste:

- 101, 201, 301, 401, 501, 601, 701: Flansch
- 102, 202, 302, 402, 502, 602, 702: Durchführung
- 103, 203, 303, 403, 503, 603, 703: Anschlussstift
- 104, 204, 304, 404, 504, 604, 704: äußeres, biokompatibles Ende des Anschlussstifts
- 105, 205, 305, 405, 505, 605, 705: inneres Ende der Anschlussstifts
- 106, 206, 306, 406, 506, 606, 706: Durchführungshülse, innere Durchführungshülse
- 207, 307, 407, 507, 607, 707: Öffnung im Flansch
- 108, 208, 308, 408, 508, 607, 708: Lotverbindung zwischen Flansch und Durchführungshülse
- 109, 209, 309, 409, 509, 609, 709: Lotverbindung zwischen Anschlusselektrode und Durchführungshülse
- 310, 410: Nagelkopfartiger Ansatz am inneren Ende des Anschlussstiftes
- 111, 211, 311, 411, 511, 611, 711: weichlötbarer Aufsatz des Anschlussstiftes
- 112, 212, 312, 412, 512, 612, 712: Fügestelle zwischen Anschlussstift und Aufsatz
- 113,213,313,413,513,613: Lotverbindung zwischen Massestift und Flansch
- 114,214,314,414,514,614,714: Masse-Anschlussstift oder Massestift oder Massepin
- 117, 217, 317, 417, 517, 617, 717: Nut im Flansch zur Aufnahme der Implantat-Gehäusehälften
- 118, 218, 318, 418, 518, 618, 718: Innenseite Lippe
- 219, 319, 419, 519, 619: Öffnung zur Aufnahme des Massestiftes
- 428: Abstufung im Flansch
- 429: Abstufung in der Durchführungshülse
- 430, 530, 630, 730: Weichlot (am Ende planiert) oder weichlötbare Beschichtung Schicht am weichlötbaren Aufsatz
- 535: "Slot" bzw. Vertiefung in der Durchführungshülse 506 zur Vergrößerung der Isolationsstrecke z. B. zwischen zwei Stiften 503 oder zwischen einem Stift 503 und dem Flansch 501
- 636, 736: Äußere Durchführungshülse
- 537: Schweißeinpassung im Flansch
- 751: Schweißpunkte bzw. -nähte 750
- 752: Hohlraum
- 753: weichlötbare und schweißbare metallische Hülse
- 754: Lötverbindung zwischen Hülse 753 und Metallisierung 755 des Kondensators 757
- 755, 765: Metallisierung des Kondensators 757
- 756, 758: Kondensatorelektrode
- 757: Keramik-Dielektrikum
- 764: Lötverbindung zwischen weichlötbarem Stift 711 und Metallisierung 765 des Kondensators 757

## Patentansprüche

1. Eine elektrische Durchführung (102, 202, 302, 402, 502, 602, 702) für ein elektromedizinisches Implantat, mit mindestens einer Durchführungshülse (106, 206, 306, 406, 506, 606, 706), einem die mindestens eine Durchführungshülse umgebenden Flansch (101, 201, 301, 401, 501, 601, 701) und mindestens einem Anschlussstift (103, 203, 303, 403, 503, 603, 703), der von der mindestens einen Durchführungshülse umgeben ist, wobei der Anschlussstift einen biokompatiblen Abschnitt (104, 204, 304, 404, 504, 604, 704) und im Inneren des Implantates einen Aufsatz (111, 211, 311, 411, 511, 611, 711) aufweist, der sich am inneren Ende des Anschlussstiftes befindet und aus Nickel, Kupfer, Palladium, Gold, Silber, Eisen oder Legierungen aus diesen Materialien besteht, **dadurch gekennzeichnet, dass** der Aufsatz im Inneren des Implantates als Stift (711) ausgebildet ist.

2. Die elektrische Durchführung nach Anspruch 1, **dadurch gekennzeichnet, dass** am Stift ein Filter, vorzugsweise ein Filterkondensator elektrisch und mechanisch verbunden ist und das Filter eine Schirmung zwischen Flansch und dem mindestens einen Anschlussstift herstellt.

3. Die elektrische Durchführung nach einem der vorgenannten Ansprüche 1 bis 2, **dadurch gekennzeichnet, dass** der Aufsatz mittels einer Fügestelle (112, 212, 312, 412, 512, 612, 712) an den biokompatiblen Abschnitt des Anschlussstiftes angebunden, insbesondere hart angelötet, angeschweißt, angecrimpt, geklemmt oder elektrisch leitfähig angeklebt, aber vorzugsweise mit Goldlot hart angelötet ist.

4. Die elektrische Durchführung nach Anspruch 3, **dadurch gekennzeichnet, dass** sich die Fügestelle (712) innerhalb der mindestens einen Durchführungshülse (706) befindet.

5. Die elektrische Durchführung nach Anspruch 1 oder 4, **dadurch gekennzeichnet, dass** die mindestens eine Durchführungshülse (106, 206, 306, 406, 506, 606, 636, 706, 736) aus Keramikmaterial, insbesondere Aluminiumoxid (Al₂O₃) besteht.

6. Die elektrische Durchführung nach Anspruch 1, **dadurch gekennzeichnet, dass** das innere Ende (310, 410) des Anschlussstifts nagelkopfförmig ausgebildet ist.

7. Die elektrische Durchführung nach Anspruch 1 oder 6, **dadurch gekennzeichnet, dass** der biokompatible Abschnitt (104, 204, 304, 404, 504, 604, 704) des Anschlussstifts aus Nb, Ta, Ti, Pt, Ir, Zr, Hf, medizinische Edelstähle, Pt/Ir oder Legierungen aus diesen Materialien, aber auch FeNi, FeNiCo, FeCr, Mo, W, Cr, FeCr, V, Al oder Legierungen mit diesen Materialien besteht.

8. Die elektrische Durchführung nach einem der vorgenannten Ansprüche, **dadurch gekennzeichnet, dass** der Anschlussstift (103, 203, 303, 403, 503, 603, 703) mit der mindestens einen Durchführungshülse (106, 206, 306, 406, 506, 606, 636, 706, 736) und/oder die mindestens eine Durchführungshülse mit dem Flansch (101, 201, 301, 401, 501, 601, 701) mittels einer Lotverbindung108, 109, 208, 209, 308, 309, 408, 409, 508, 509, 608, 609, 708, 709) hermetisch dicht verbunden sind, wobei die Lotverbindung vorzugsweise mittels eines Glaslotes erfolgt.

9. Die elektrische Durchführung nach Anspruch 5, **dadurch gekennzeichnet, dass** der mindestens eine Anschlussstift (103, 203, 303, 403, 503, 603, 703) mit einer äußeren und eine inneren Durchführungshülse (106, 206, 306, 406, 506, 606, 636, 706, 736) und die äußere und innere Durchführungshülse mit dem Flansch (101, 201, 301, 401, 501, 601, 701) mittels einer als Glaspfropf ausgebildeten Lotverbindung hermetisch dicht verbunden ist, wobei der Glaspfropf durch einen von Flansch, äußerer und innerer Durchführungshülse umschlossenen Hohlraum begrenzt ist.

10. Verfahren zur Herstellung einer Durchführung nach einem der Ansprüche 1 bis 9, **dadurch gekennzeichnet, dass** während der Erzeugung des Glaslot-Pfropfs der Anschlussstift (103, 203, 303, 403, 503, 603, 703 mittels einer Wärmesenke gekühlt wird.

11. Verwendung einer Durchführung nach einem der Ansprüche 1 bis 9, **dadurch gekennzeichnet, dass** ein innenseitiges Weichlöten am Anschlussstift (103, 203, 303, 403, 503, 603, 703 mittels eines Reflow-Verfahrens ausgeführt wird.

## Claims

1. An electrical feedthrough (102,202, 302,402, 502, 602, 702) for an electromedical implant, having at least one bushing (106, 206, 306, 406, 506, 606, 706), a flange (101, 201, 301, 401, 501, 601, 701) surrounding the at least one bushing and at least one connector pin (103, 203, 303, 403, 503, 603, 703), which is surrounded by the at least one bushing,
the connector pin having a biocompatible section (104, 204, 304, 404, 504, 604, 704) and an attachment (111, 211, 311, 411, 511, 611, 711) inside the implant, which attachment is located at the inner end of the connector pin and is made of nickel, copper, palladium, gold, silver, iron or alloys made of these materials, **characterized in that** the attachment is designed as a pin (711) inside the implant.

2. The electrical feedthrough according to claim 1, **characterized in that** a filter, preferably a filter capacitor, is electrically and mechanically connected to the pin and the filter creates a shielding between the flange and the at least one connector pin.

3. The electrical feedthrough according to any one of the preceding claims 1 to 2, **characterized in that** the attachment is connected to the biocompatible section of the connector pin by means of a joint (112, 212, 312, 412, 512, 612, 712), particularly hard soldered, welded, crimped, clamped or glued in an electrically conductive manner, but is preferably hard soldered with gold solder.

4. The electrical feedthrough according to claim 3, **characterized in that** the joint (712) is located within the at least one bushing (706).

5. The electrical feedthrough according to claim 1 or 4, **characterized in that** the at least one bushing (106, 206, 306, 406, 506, 606, 636, 706, 736) is made of ceramic material, particularly aluminum oxide (Al₂O₃).

6. The electrical feedthrough according to claim 1, **characterized in that** the inner end (310, 410) of the connector pin is designed in the shape of a nail head.

7. The electrical feedthrough according to claim 1 or 6, **characterized in that** the biocompatible section (104, 204, 304, 404, 504, 604, 704) of the connector pin is made of Nb, Ta, Ti, Pt, Ir, Zr, Hf, medical stainless steels, Pt/Ir or alloys made of these materials, but also FeNi, FeNiCo, FeCr, Mo, W, Cr, FeCr, V, Al or alloys with these materials.

8. The electrical feedthrough according to any one of the preceding claims, **characterized in that** the connector pin (103, 203, 303, 403, 503, 603, 703) is hermetically sealed to the at least one bushing (106, 206, 306, 406, 506, 606, 636, 706, 736) and/or the at least one bushing is hermetically sealed to the flange (101, 201, 301, 401, 501, 601, 701) by means of a solder connection (108, 109, 208, 209, 308, 309, 408, 409, 508, 509, 608, 609, 708, 709), wherein the solder connection is preferably made by means of a glass solder.

9. The electrical feedthrough according to claim 5, **characterized in that** the at least one connector pin (103, 203, 303, 403, 503, 603, 703) is hermetically sealed to an outer and an inner bushing (106, 206, 306, 406, 506, 606, 636, 706, 736), and the outer and inner bushing is hermetically sealed to the flange (101, 201, 301, 401, 501, 601, 701) by means of a solder connection designed as a glass plug, wherein the glass plug is delimited by a cavity enclosed by a flange, an outer and an inner bushing.

10. A method for producing a feedthrough according to any one of claims 1 to 9, **characterized in that** the connector pin (103, 203, 303, 403, 503, 603, 703) is cooled by means of a heat sink while the glass solder plug is being produced.

11. A use of a feedthrough according to any one of claims 1 to 9, **characterized in that** soft soldering on the inside of the connector pin (103, 203, 303, 403, 503, 603, 703) is carried out by means of a reflow process.

## Revendications

1. Traversée électrique (102, 202, 302, 402, 502, 602, 702) destinée à un implant électromédical, avec au moins un manchon de traversée (106, 206, 306, 406, 506, 606, 706), une bride (101, 201, 301, 401, 501, 601, 701) entourant l'au moins un manchon de traversée et au moins une broche de raccordement (103, 203, 303, 403, 503, 603, 703) qui est entourée par l'au moins un manchon de traversée,
dans laquelle la broche de raccordement présente un segment (104, 204, 304, 404, 504, 604, 704) biocompatible et une rehausse (111, 211, 311, 411, 5011, 611, 711) à l'intérieur de l'implant qui se situe à l'extrémité intérieure de la broche de raccordement et est constituée de nickel, de cuivre, de palladium, d'or, d'argent, de fer ou d'alliages à base de ces matériaux, **caractérisé en ce que** la rehausse à l'intérieur de l'implant est conçue sous forme de broche (711).

2. Traversée électrique selon la revendication 1, **caractérisée en ce qu'**un filtre, de préférence, un condensateur filtre est relié électriquement et mécaniquement sur le filtre, et le filtre établit un blindage entre la bride et l'au moins une broche de raccordement.

3. Traversée électrique selon l'une des revendications précitées 1 à 2, **caractérisée en ce que** la rehausse est attachée, en particulier, brasée, soudée, sertie, serrée ou collée de manière électriquement conductrice sur le segment biocompatible de la broche de raccordement au moins d'un point d'assemblage (112, 212, 312, 412, 512, 612, 712), mais est de préférence sodé avec un produit de soudage en or.

4. Traversée électrique selon la revendication 3, **caractérisée en ce que** le point d'assemblage (712) se situe à l'intérieur de l'au moins un manchon de traversée (706).

5. Traversée électrique selon la revendication 1 ou selon la revendication 4, **caractérisée en ce que** l'au moins un manchon de traversée (106, 206, 306, 406, 506, 706, 736) est constituée d'un matériau céramique, notamment d'oxyde d'aluminium (Al₂O₃).

6. Traversée électrique selon la revendication 1, **caractérisée en ce que** l'extrémité intérieure (310, 410) de la broche de raccordement est conçue en forme de tête d'épingle.

7. Traversée électrique selon la revendication 1 ou la revendication 6, **caractérisée en ce que** le segment (104, 204, 304, 404, 504, 604, 704) biocompatible de la broche de raccordement est constitué de Nb, Ta, Ti, Pt, Ir, Zr, Hf, de métaux nobles pour le domaine médical, de Pt/Ir ou d'alliages de ces matériaux, mais également de FeNi, FeNiCo, FeCr, Mo, W, Cr, FeCr, V, Al ou d'alliage avec ces matériaux.

8. Traversée électrique selon l'une des revendications précédemment citées, **caractérisée en ce que** la broche de raccordement (103, 203, 303, 403, 503, 603, 703) est reliée de manière étanche et hermétique avec l'au moins un manchon de traversée (106, 206, 306, 406, 506, 606, 636, 706, 736) et/ou l'au moins un manchon de traversée est reliée avec la bride (101, 201, 301, 401, 501, 601, 701) au moyen d'une liaison soudée 108, 109, 208, 209, 308,, 309, 408, 409, 508, 509, 608, 609, 708, 709, la liaison soudée ayant de préférence lieu au moyen d'un verre de soudure.

9. Traversée électrique selon la revendication 5, **caractérisée en ce que** l'au moins une broche de raccordement (103, 203, 303, 403, 503, 603, 703) est reliée de manière étanche et hermétique avec un manchon de traversée extérieur et un manchon de traversée intérieur (106, 206, 306, 406, 506, 606, 636, 706, 736) et/ou le manchon de traversée intérieur et le manchon de traversée extérieur sont reliés avec la bride (101, 201, 301, 401, 501, 601, 701) au moyen d'une liaison soudée conçue sous forme d'un bouchon en verre, le bouchon en verre étant délimité par une cavité entourée par la bride, le manchon de traversée extérieur et le manchon de traversée intérieur.

10. Procédé de fabrication d'une traversée selon l'une des revendications 1 à 9, **caractérisé en ce que**, pendant l'élaboration du bouchon en verre de soudure, la broche de raccordement (103, 203, 303, 403, 503, 603, 703 est refroidie au moyen d'un dissipateur de chaleur.

11. Utilisation d'une traversée selon l'une des revendications 1 à 9, **caractérisée en ce qu'**une brasure tendre est exécutée sur le coté intérieur sur la broche de raccordement (103, 203, 303, 403, 503, 603, 703 au moyen d'un procédé de refusion.
